(19) Europäisches Patentamt
European Patent Office
Office européen des brevets

(11) EP 2 670 378 B1

(12) EUROPEAN PATENT SPECIFICATION

(45) Date of publication and mention of the grant of the patent:
**16.08.2017 Bulletin 2017/33**

(21) Application number: **12701723.4**

(22) Date of filing: **23.01.2012**

(51) Int Cl.:
*A61K 8/25* (2006.01) *A61K 8/29* (2006.01)
*A61Q 17/00* (2006.01) *A61K 8/06* (2006.01)
*A61Q 17/04* (2006.01) *A61K 8/02* (2006.01)

(86) International application number:
**PCT/EP2012/050958**

(87) International publication number:
**WO 2012/104161 (09.08.2012 Gazette 2012/32)**

# (54) OIL-IN-WATER EMULSION COMPRISING A MIXTURE OF SPHERICAL AND NON-SPHERICAL SCREENING PARTICLES OF COMPOSITE MATERIAL

ÖL-IN-WASSER-EMULSION MIT EINEM GEMISCH VON KUGELFÖRMIGEN UND NICHT-KUGELFÖRMIGEN SCREENING-PARTIKELN EINES VERBUNDWERKSTOFFS

ÉMULSION HUILE DANS EAU COMPRENANT UN MÉLANGE DE PARTICULES FILTRANTES SPHÉRIQUES ET NON SPHÉRIQUES DE MATIÈRE COMPOSITE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.02.2011 FR 1150918**
**11.02.2011 US 201161441844 P**

(43) Date of publication of application:
**11.12.2013 Bulletin 2013/50**

(73) Proprietor: **L'Oréal**
**75008 Paris (FR)**

(72) Inventors:
- **L'ALLORET, Florence**
  **F-75014 Paris (FR)**
- **WILLIEN, Maud**
  **F-94400 Vitry Sur Seine (FR)**

(74) Representative: **Miszputen, Laurent**
**L'Oréal**
**Service DIPI**
**9 Rue Pierre Dreyfus**
**92110 Clichy (FR)**

(56) References cited:
**JP-A- 2007 302 647 US-A1- 2008 188 574**

- **Anonymous: "Stradivarious" foundation (colour)", Creations Couleurs Guide Formulary, no. 1406-2/2010 February 2010 (2010-02), XP002659379, Retrieved from the Internet: URL:http://www.creationscouleurs.com/formulations/sun/spf_foundations/1406_2_2010StradivariusFoundationColour.pdf [retrieved on 2011-09-05]**
- **Anonymous: "Eospoly", Creations Couleurs Application Sheet, January 2008 (2008-01), XP002659381, Retrieved from the Internet: URL:http://www.creationscouleurs.com/as/as_eospoly_web.pdf [retrieved on 2011-09-05]**
- **ANONYMOUS: "Suncare compositions with new cosmetic raw materials", IP.COM JOURNAL, IP.COM INC., WEST HENRIETTA, NY, US, 2 March 2010 (2010-03-02), XP013137210, ISSN: 1533-0001**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 670 378 B1

## Description

The present invention relates to a composition in the form of an oil-in-water emulsion containing a mixture of spherical and non-spherical screening composite particles. This composition is for topical use and is more particularly intended for the photoprotection of the skin and/or hair against ultraviolet (UV) radiation.

It is known that light radiation with wavelengths of between 280 nm and 400 nm permits tanning of the human epidermis and that light rays with wavelengths more particularly between 280 and 320 nm, known as UV-B rays, cause skin burns and erythema which can harm the development of a natural tan.

For these reasons, and also for aesthetic reasons, there is constant demand for means for controlling this natural tanning in order thus to control the colour of the skin; this UV-B radiation should thus be screened out.

It is also known that UV-A rays, with wavelengths between 320 and 400 nm, which cause tanning of the skin, are liable to induce adverse changes therein, in particular in the case of sensitive skin or skin that is continually exposed to solar radiation. UV-A rays cause in particular a loss of elasticity of the skin and the appearance of wrinkles leading to premature ageing of the skin.

Thus, for aesthetic and cosmetic reasons, for instance conservation of the skin's natural elasticity, people increasingly wish to control the effect of UV-A rays on their skin. It is thus desirable also to screen out UV-A radiation.

For the purpose of protecting the skin and keratin materials against UV radiation, antisun compositions comprising organic screening agents that are active in the UV-A range and in the UV-B range are generally used.

There are many cosmetic products comprising one or more inorganic and/or organic UV screening agents. In particular, the cosmetic products intended to be applied to the skin often comprise fine $TiO_2$ particles to protect the skin from UV rays.

These fine metal oxide particles generally have a mean elementary particle size of less than or equal to 100 nm, preferably between 5 nm and 100 nm, preferably between 10 nm and 100 nm, and preferentially between 15 and 50 nm.

One of the major drawbacks of inorganic screening agents lies in the fact that conventional antisun formulations based on metal oxide pigments result, after application to the skin, in an uneven, inhomogeneous or even coarse distribution of said pigments on this skin, which may be detrimental to the quality of the overall photoprotective effect desired. This poor distribution of the screening metal oxide pigments that is observed at the surface of the skin is often linked to the fact that there is, in the initial composition itself (before application), a substantial lack of homogeneity (poor dispersion of the pigment in its support).

Another drawback of antisun compositions based on inorganic screening agents and more particularly based on titanium oxide $TiO_2$ is that, once applied to the skin in the form of a film, they create, on the latter, a whitening effect which is cosmetically undesirable and is generally poorly appreciated by users. This effect is even more pronounced when the concentration of mineral screening agents in the composition is high. To avoid this problem, it would of course be possible to use reduced amounts of inorganic screening agent(s), but the resulting compositions, which would certainly result in films exhibiting an acceptable transparency on the skin, would then no longer offer suitable protection in the UV range, which greatly limits the advantage of such an option.

Patent applications FR 2882371, WO 2006/083326 and WO 98/37964 describe various processes for manufacturing composite particles formed from a material comprising nanoparticles of metal oxides, such as titanium dioxide.

Application WO 2006/061835 describes compositions comprising spherical composites based on a metal oxide and on a hydrophobic polymer.

Known in the cosmetic field are application EP 1 388 550, which targets the use of composite particles comprising a core formed of a metal oxide coated with a silicone or fluoro compound and the use thereof as a photoprotective cosmetic composition, and application WO 98/22539, which describes a sunscreen containing a particle of silicon and/or of another solid compound in which the silicon is in stoichiometric excess, the said particle having a mean diameter of less than 0.12 μm and being covered with a layer of oxide having a thickness ranging from 0.001 to 0.3 μm.

Antisun formulations that may contain as screening system spherical particles of composite material with a mean size of between 2 and 7 μm, $TiO_2$ encapsulated in spherical silica particles are known, such as those sold under the name Sunsil T50 by Sunjin Chemical or Eospoly TR sold by the company Créations Couleurs. These screening materials have the drawback of leading to formulations whose stability and cosmetic qualities, such as the spreadability, are still insufficient.

Patent application US 2008/188 574 discloses antisun oil/water emulsions comprising organic screening agents, spherical particles of composite material of $TiO_2$ encapsulated in spherical silica particles, such as those sold under the name Sunsil TIN 40 by Sunjin Chemical combined with a clay of the Veegum type. These compositions are not entirely satisfactory from the point of view of the stability or the cosmetic qualities.

Patent application JP 2007-302647 and the following documentations disclose water-in-oil and oil-in-water emulsions comprising as screening agent particles of composite material based on $TiO_2$ combined with a clay such as Micapoly Foundation Creations Couleurs- Guide Formulary No. 1210-1/2010 January 2010;

URL: ;http://www.creationcouleurs.com/formulations/sun /spf_foundations/1201_1_2010micapolyfoundation.pdf Stradivarius Foundation Creations Couleurs- Guide Formulary No. 1406-2/2010 February 2010; URL: ;http://www.creationcouleurs.com/formulations/sun /spf_foundations/1406_2_2010stradivariusFoundationColour.pdf;

Sun Protection Fluid Creations Couleurs- Guide Formulary No. 2804-1/2008 January 2008; URL: ;http://www.creationcouleurs.com/formulations/sun/low_spf_ /2804_1_2008mSunProtectionFluidW_0. pdf Eospoly Protection Sun Cream Creations Couleurs- Guide Formulary No. 3103-1/2008 January 2008; URL: ;http://www.creationcouleurs.com/formulations/sun /low_spf_/3103_1_2008meospolysuncr.pdf.

These emulsions are not entirely satisfactory from the point of view of the cosmetic qualities, due to the fact that they have a tendency to give the skin a greasy, tacky feel.

There is thus still a need for UV sun protection compositions that afford efficient photoprotection and that do not have the drawbacks presented above.

Unexpectedly and advantageously, the inventors have shown that this need could be met by means of the compositions according to the present invention.

A first subject of the present invention concerns a composition in the form of an oil-in-water emulsion containing, in a cosmetically acceptable medium, a mixture of composite particles comprising:

a) at least spherical composite particles A with a mean size of between 0.1 and 30 μm comprising a matrix and an inorganic UV-screening agent, the content of inorganic screening agent in a particle ranging from 1% to 70% by weight,

b) at least non-spherical composite particles B with a mean size of between 0,3 and 30 μm comprising a matrix and an inorganic UV-screening agent, the content of inorganic screening agent in a particle ranging from 1% to 70% by weight.

The composition according to the present invention is effective in photoprotection. Moreover, the composition has a homogeneous appearance, in particular on the microscopic scale and it does not generate a white deposit when it is applied to the skin.

The following description and examples present other advantages, aspects and properties of the present invention.

## Definitions

The following definitions are used in the present text.

The compositions according to the present invention are photoprotective compositions intended to screen UV radiation; these compositions are also known as anti-sun compositions or sun protection compositions.

The term "cosmetically acceptable" means compatible with the skin and/or its integuments or mucous membranes, having a pleasant colour, odour and feel and not causing any unacceptable discomfort (stinging, tautness or redness) liable to discourage the consumer from using this composition.

The term "oil-in-water emulsion" means any macroscopically homogeneous composition comprising a continuous aqueous phase and an oily phase dispersed in the said aqueous phase in the form of droplets.

The expression "mean size" of the particles is understood to mean the parameter D[4,3] measured using a "Mastersizer 2000" particle size analyser (Malvern). The light intensity scattered by the particles as a function of the angle at which they are lit is converted to size distribution according to Mie theory. The parameter D[4,3] is measured; this is the mean diameter of the sphere having the same volume as the particle. For a spherical particle, reference will often be made to the "mean diameter".

The term "mean elementary size" means the size of non-aggregated particles.

The term "spherical" means that the particle has a sphericity index, i.e. the ratio between its largest diameter and its smallest diameter, of less than 1.2.

The term "non-spherical" refers to particles in three dimensions (length, width, thickness or height) for which the ratio of the longest dimension to the shortest dimension is greater than 1.2. The dimensions of the particles of the invention are evaluated by scanning electron microscopy and image analysis. They comprise particles of parallelepipedal shape (rectangular or square surface), discoid shape (circular surface) or ellipsoid shape (oval surface), characterized by three dimensions: a length, a width and a height. When the shape is circular, the length and the width are identical and correspond to the diameter of a disc, whereas the height corresponds to the thickness of the disc. When the surface is oval, the length and the width correspond, respectively, to the large axis and the small axis of an ellipse and the height corresponds to the thickness of the elliptic disc formed by the platelet. When it is a parallelepiped, the length and the width may be of identical or different dimensions: when they are of the same dimension, the shape of the surface of the parallelepiped is a square; in the contrary case, the shape is rectangular. As regards the height, it corresponds to the

thickness of the parallelepiped.

## SCREENING COMPOSITE PARTICLES

The spherical and non-spherical screening composite particles used according to the present invention comprise a matrix and an inorganic UV-screening agent. The matrix comprises one or more organic and/or inorganic materials.
The inorganic UV-screening agent is generally chosen from metal oxides, preferably titanium, zinc or iron oxides or mixtures thereof and more particularly from titanium dioxide $TiO_2$.
These metal oxides may be in the form of particles with a mean size generally of less than 0.2 μm. Advantageously, the metal oxide particles used have a mean elementary size of less than or equal to 0.1 μm.
These metal oxides may also be in the form of layers, preferably multilayers with a mean thickness generally of less than 0.2 μm.
According to a first variant, the composite particles contain a matrix comprising an organic and/or inorganic material, in which matrix particles of inorganic UV-screening agent are included. According to this embodiment, the matrix has inclusions and particles of inorganic UV-screening agent are placed in the inclusions of the matrix.
According to a second variant, the composite particles contain a matrix made of an organic and/or inorganic material, which matrix is covered with at least one layer of inorganic UV-screening agent which may be connected to the matrix with the aid of a binder.
According to a third variant, the composite particles contain an inorganic UV-screening agent covered with at least one layer of an organic and/or inorganic material.
The matrix may also be formed from one or more organic or inorganic materials. It may then be a continuous phase of materials such as an alloy, i.e. a continuous phase in which the materials can no longer be dissociated, or a discontinuous phase of materials, for example constituted of an organic or inorganic material covered with a layer of another different organic or inorganic material.
According to one variant, in particular when the spherical composite particles comprise a matrix covered with a layer of UV-screening agent, the composite particles may furthermore be covered with an additional coating, in particular chosen from biodegradable or biocompatible materials, lipid materials, for instance surfactants or emulsifiers, polymers, and oxides.

## Spherical composite particles A

The inorganic materials that may be used in the matrix of the spherical composite particles according to the present invention may be chosen from the group formed by glass, silica and aluminium oxide, and mixtures thereof.
The organic materials that may be used to form the matrix are chosen from the group formed by poly(meth)acrylates, polyamides, silicones, polyurethanes, polyethylenes, polypropylenes, polystyrenes, polycaprolactams, polysaccharides, polypeptides, polyvinyl derivatives, waxes, polyesters and polyethers, and mixtures thereof.
Preferably, the matrix of the spherical composite particle contains a material or mixture of materials chosen from:

- $SiO_2$,
- polymethyl methacrylate,
- copolymers of styrene and of a C1/C5 alkyl (meth)acrylate derivative,
- polyamides, such as nylon.

The composite particles in spherical form are characterized by a mean diameter between 0.1 μm and 30 μm, preferably between 0.2 μm and 20 μm and more preferably between 0.3 μm and 10 μm, advantageously between 0.5 μm and 10 μm.
According to a first variant, the spherical composite particles contain a matrix comprising an organic and/or inorganic material, in which matrix particles of inorganic UV-screening agent are included.
According to this first variant, the particles of inorganic UV-screening agent are characterized by a mean elementary size generally less than 200 nm. Advantageously, the metal oxide particles used have a mean elementary size of less than or equal to 0.1 μm.
As composite particles corresponding to this variant, mention may be made of the products Sunsil TIN 50 and Sunsil TIN 40 sold by the company Sunjin Chemical. These spherical composite particles with a mean size between 2 and 7 μm are formed from $TiO_2$ encapsulated in a silica matrix.
Mention may also be made of the following particles:

- spherical composite particles with a mean size between 4 and 8 μm, containing $TiO_2$ and $SiO_2$, having the trade name Eospoly TR sold by the company Créations Couleurs,

- composite particles containing $TiO_2$ and a styrene/alkyl acrylate copolymer matrix sold under the name Eospoly UV TR22 HB 50 by the company Créations Couleurs,
- composite particles containing $TiO_2$ and ZnO and a PMMA matrix, having the trade name Sun PMMA-T50 sold by the company Sunjin Chemical.

According to a second variant, the spherical composite particles contain a matrix made of an organic and/or inorganic material, covered with at least one layer of inorganic UV-screening agent connected to the matrix by means of a binder.
According to this second variant, the mean thickness of the layer of inorganic UV-screening agent is generally between 0.001 and 0.2 μm and preferably between 0.01 and 0.1 μm.
The spherical composite particles used according to the invention have a size of between 0.1 and 30 μm, preferably between 0.3 and 20 μm and even more preferentially between 0.5 and 10 μm.
Among the composite particles that may be used according to the invention, mention may also be made of spherical composite particles containing $TiO_2$ and $SiO_2$, having the trade name STM ACS-0050510, supplied by the company JGC Catalysts and Chemical.
According to a third variant, the spherical composite particles contain an inorganic UV-screening agent covered with at least one layer of an organic and/or inorganic material. According to this third variant, the particles of inorganic UV-screening agent are characterized by a mean elementary size generally of between 0.001 and 0.2 μm. Advantageously, the metal oxide particles used have a mean elementary size between 0.01 and 0.1 μm.
The spherical composite particles used according to the invention have a size of between 0.1 and 30 μm, preferably between 0.3 and 20 μm and even more preferentially between 0.5 and 10 μm.

**<u>Non-spherical composite particles B</u>**

The organic materials that may be used to form the matrix of the non-spherical screening particles are chosen from the group formed by polyamides, silicones, polysaccharides, polyvinyl derivatives, waxes and polyesters, and mixtures thereof.
Among the organic materials that may be used, mention is preferably made of:

- triethoxycaprylylsilane,
- ethylene/vinyl acetate copolymers.

The inorganic materials that may be used in the matrix of the non-spherical composite particles are chosen from the group formed by mica, synthetic mica, talc, silica, aluminium oxide, boron nitride, kaolin, hydrotalcite, mineral clays and synthetic clays, and mixtures thereof. Preferably, these inorganic materials are chosen from:

- silica,
- talc,
- mica,
- alumina.

The non-spherical composite particles of the invention are characterized by three dimensions, namely:

- the smallest is greater 0.3 μm and better still greater than 0.5 μm,
- the largest is less than 30 μm, preferably 20 μm and better still 10 μm.

The ratio of the largest to the smallest dimension is greater than 1.2.
The dimensions of the particles of the invention are evaluated by scanning electron microscopy and image analysis.
The non-spherical screening composite particles that may be used according to the invention will preferably be platelet-shaped.
The term "platelet-shaped" means parallelepipedal-shaped.
They may be smooth, rough or porous.
The platelet-shaped composite particles preferably have a mean thickness of between 0.1 and 10 μm, the mean length is generally between 0.5 and 30 μm and the mean width is between 0.5 and 30 μm.
The thickness is the smallest of the dimensions, the width is the medium dimension, and the length is the longest of the dimensions.
According to a first variant, the composite particles contain a matrix comprising an organic and/or inorganic

material, in which matrix particles of inorganic UV-screening agent are included.

According to this first variant, the particles of inorganic UV-screening agent are characterized by a mean elementary size generally less than 0.2 μm. Advantageously, the metal oxide particles used have a mean elementary size of less than or equal to 0.1 μm.

According to a second variant, the composite particles contain a matrix made of an organic and/or inorganic material, covered with at least one layer of inorganic UV-screening agent connected to the matrix by means of a binder.

According to this second variant, the mean thickness of the layer of inorganic UV-screening agent is generally about ten nanometres. The mean thickness of the layer of inorganic UV-screening agent is advantageously between 0.001 and 0.2 μm and preferably between 0.01 and 0.2 μm.

According to a third variant, the non-spherical composite particles contain an inorganic UV-screening agent covered with at least one layer of an organic and/or inorganic material. According to this third variant, the particles of inorganic UV-screening agent are characterized by a mean elementary size generally of between 0.001 and 0.2 μm. Advantageously, the metal oxide particles used have a mean elementary size between 0.01 and 0.1 μm.

The non-spherical composite particles used according to the invention have a size of between 0,3 and 30 μm and preferably between 0.5 and 10 μm.

Preferably, the inorganic UV-screening agent used in the composite particle is chosen from metal oxides, in particular from titanium, zinc or iron oxides and more particularly titanium dioxide ($TiO_2$).

Preferably, the matrix of the composite particle contains a material or mixture of materials chosen from:

- $SiO_2$,
- alumina,
- mica,
- an alumina/triethoxycaprylylsilane mixture,
- talc,
- Nylon.

More preferably, the matrix of the composite particle is formed from a material or mixture of materials chosen from:

- alumina,
- an alumina/triethoxycaprylylsilane mixture,
- talc,
- silica,
- mica.

Among the composite particles that may be used according to the invention, mention may also be made of the following particles:

- composite particles containing $TiO_2$ and an alumina matrix, of trade name Matlake OPA, sold by the company Sensient LCW,
- composite particles containing $TiO_2$ and an alumina/triethoxycaprylylsilane matrix, of trade name Matlake OPA AS, sold by the company Sensient LCW,
- composite particles containing ultrafine $TiO_2$ particles deposited on the surface of talc platelets, of trade name TTC 30, sold by the company Miyoshi Kasei,
- composite particles containing ultrafine $TiO_2$ particles deposited on the surface of talc platelets, of trade name Silseem Mistypearl Yellow, sold by the company Nihon Koken Kogyo (NKK).

The content of mixture of composite particles in the composition according to the invention ranges from 1% to 70%, preferably from 1.5% to 45% and preferably from 2% to 20% by weight relative to the total weight of the cosmetic composition.

According to one particularly preferred form, the oily phase (anhydrous phase) of the emulsion comprises at least one polar oil.

The term "anhydrous medium" means any support comprising less than 3% by weight of water, or less than 1% by weight of water, or more particularly less than 0.5% by weight of water relative to the total weight of the composition, or even is free of water.

**Polar oils**

The term "polar oil" means any lipophilic compound having, at 25°C, a solubility parameter $\delta_d$ characteristic of dispersive interactions of greater than 16 and a solubility parameter $\delta_p$ characteristic of polar interactions strictly greater than 0. The solubility parameters $\delta_d$ and $\delta_p$ are defined according to the Hansen classification. For example, these polar oils may be chosen from esters, triglycerides and ethers.
The definition and calculation of the solubility parameters in the Hansen three-dimensional solubility space are described in the article by C.M. Hansen: "The three dimensional solubility parameters", J. Paint Technol. 39, 105 (1967).
According to this Hansen space:

- $\delta_D$ characterizes the London dispersion forces derived from the formation of dipoles induced during molecular impacts;
- $\delta_p$ characterizes the Debye interaction forces between permanent dipoles and also the Keesom interaction forces between induced dipoles and permanent dipoles;
- $\delta_h$ characterizes the specific interaction forces (such as hydrogen bonding, acid/base, donor/acceptor, etc.); and
- $\delta_a$ is determined by the equation: $\delta_a = (\delta_p^2 + \delta_h^2)^{½}$.

The parameters $\delta_p$, $\delta_h$, $\delta_D$ and $\delta_a$ are expressed in $(J/cm^3)^{½}$.
The polar oil may be a volatile or non-volatile hydrocarbon-based oil.
These oils may be of plant, mineral or synthetic origin.
The term "polar hydrocarbon-based oil" means an oil formed essentially from, or even constituted by, carbon and hydrogen atoms, and optionally oxygen and nitrogen atoms, and not containing any silicon or fluorine atoms. It may contain alcohol, ester, ether, carboxylic acid, amine and/or amide groups.
Preferentially, the polar oil according to the invention has a surface tension of greater than 10 mN/m at 25°C and at atmospheric pressure.
The surface activity is measured by static tensiometry using the Du Noüy ring.
The principle of the measurement is as follows (measurement carried out at 25°C, at atmospheric pressure):

The weight of the ring is neutralized by a tare. The ring is completely immersed in the liquid to be evaluated, then withdrawn very slowly until the force reaches its maximum. From this maximum force $F_{max}$, the surface tension is calculated according to the equation:

$$\sigma = F_{max} / 4\pi R\, f_{corr}\, (r,\ R, \rho)$$

with $f_{corr}$: correction factor of the ring depending on the geometry of the ring and the density p.

The parameters r and R respectively denote the internal and external radii of the ring.
According to a first embodiment, the polar oil may be a non-volatile oil. In particular, the non-volatile polar oil may be chosen from the list of oils below, and mixtures thereof:

- hydrocarbon-based polar oils such as phytostearyl esters, such as phytostearyl oleate, phytostearyl isostearate and lauroyl/octyldodecyl/phytostearyl glutamate (Ajinomoto, Eldew PS203), triglycerides consisting of fatty acid esters of glycerol, in particular the fatty acids of which may have chain lengths ranging from $C_4$ to $C_{36}$, and especially from $C_{18}$ to $C_{36}$, these oils possibly being linear or branched, and saturated or unsaturated; these oils may especially be heptanoic or octanoic triglycerides, wheatgerm oil, sunflower oil, grapeseed oil, sesame seed oil (820.6 g/mol), corn oil, apricot oil, castor oil, shea oil, avocado oil, olive oil, soybean oil, sweet almond oil, palm oil, rapeseed oil, cottonseed oil, hazelnut oil, macadamia oil, jojoba oil, alfalfa oil, poppy oil, pumpkin oil, marrow oil, blackcurrant oil, evening primrose oil, millet oil, barley oil, quinoa oil, rye oil, safflower oil, candlenut oil, passionflower oil or musk rose oil; shea butter; or alternatively caprylic/capric acid triglycerides, for instance those sold by the company Stéarineries Dubois or those sold under the names Miglyol 810®, 812® and 818® by the company Dynamit Nobel;
- synthetic ethers containing from 10 to 40 carbon atoms, such as dicaprylyl ether;
- hydrocarbon-based esters of formula RCOOR' in which RCOO represents a carboxylic acid residue comprising from 2 to 40 carbon atoms, and R' represents a hydrocarbon-based chain containing from 1 to 40 carbon atoms, such as cetostearyl octanoate, isopropyl alcohol esters, such as isopropyl myristate or isopropyl palmitate, ethyl palmitate, 2-ethylhexyl palmitate, isopropyl stearate or isostearate, isostearyl isostearate, octyl stearate, diisopropyl adipate, heptanoates, and especially isostearyl heptanoate, alcohol or polyalcohol octanoates, decanoates or ricinoleates, for instance propylene glycol dioctanoate, cetyl octanoate, tridecyl octanoate, 2-ethylhexyl 4-diheptanoate and palmitate, alkyl benzoate, polyethylene glycol diheptanoate, propylene glycol 2-diethyl hexanoate, and mixtures thereof, $C_{12}$ to $C_{15}$ alcohol benzoates, hexyl laurate, neopentanoic acid esters, for instance isodecyl neopentanoate, isotridecyl neopentanoate, isostearyl neopentanoate and 2-octyldodecyl neopentanoate, isononanoic acid esters, for instance isononyl isononanoate, isotridecyl isononanoate and octyl isononanoate, oleyl erucate, isopropyl lauroyl sarcosinate, diisopropyl sebacate, isocetyl stearate, isodecyl neopentanoate, isostearyl behenate, and myristyl myristate;

- polyesters obtained by condensation of an unsaturated fatty acid dimer and/or trimer and of diol, such as those described in patent application FR 0 853 634, in particular such as dilinoleic acid and 1,4-butanediol. Mention may especially be made in this respect of the polymer sold by Biosynthis under the name Viscoplast 14436H (INCI name: dilinoleic acid/butanediol copolymer), or else copolymers of polyols and of dimer diacids, and esters thereof, such as Hailuscent ISDA;
- polyol esters and pentaerythritol esters, for instance dipentaerythrityl tetrahydroxystearate/tetraisostearate;
- fatty alcohols containing from 12 to 26 carbon atoms, for instance octyldodecanol, 2-butyloctanol, 2-hexyldecanol, 2-undecylpentadecanol and oleyl alcohol;
- $C_{12}$-$C_{22}$ higher fatty acids, such as oleic acid, linoleic acid and linolenic acid, and mixtures thereof;
- fluorinated oils which are optionally partially hydrocarbon-based and/or silicone-based;
- fatty acids containing from 12 to 26 carbon atoms, for instance oleic acid;
- dialkyl carbonates, the two alkyl chains possibly being identical or different, such as dicaprylyl carbonate sold under the name Cetiol CC by Cognis; and
- non-volatile oils of high molecular mass, for example between 400 and 10 000 g/mol, in particular between 650 and 10 000 g/mol, for instance:

  i) vinylpyrrolidone copolymers such as the vinylpyrrolidone/1-hexadecene copolymer, Antaron V-216 sold or manufactured by the company ISP (MW = 7300 g/mol),
  ii) esters such as:

    a) linear fatty acid esters with a total carbon number ranging from 35 to 70, for instance pentaerythrityl tetrapelargonate (MW = 697.05 g/mol),
    b) hydroxylated esters such as polyglycerol-2 triisostearate (MW = 965.58 g/mol),
    c) aromatic esters such as tridecyl trimellitate (MW = 757.19 g/mol), $C_{12}$-$C_{15}$ alcohol benzoate, 2-phenylethyl benzoate and butyloctyl salicylate,
    d) esters of $C_{24}$-$C_{28}$ branched fatty acids or fatty alcohols such as those described in patent application EP-A-0 955 039, and especially triisoarachidyl citrate (MW = 1033.76 g/mol), pentaerythrityl tetraisononanoate (MW = 697.05 g/mol), glyceryl triisostearate (MW = 891.51 g/mol), glyceryl tris(2-decyl)tetradecanoate (MW = 1143.98 g/mol), pentaerythrityl tetraisostearate (MW = 1202.02 g/mol), polyglyceryl-2 tetraisostearate (MW = 1232.04 g/mol) or else pentaerythrityl tetrakis(2-decyl)tetradecanoate (MW = 1538.66 g/mol),
    e) esters and polyesters of dimer diol and of monocarboxylic or dicarboxylic acid, such as esters of dimer diol and of fatty acid and esters of dimer diol and of dimer dicarboxylic acid, such as Lusplan DD-DA5® and Lusplan DD-DA7® sold by the company Nippon Fine Chemical and described in patent application US 2004-175 338, the content of which is incorporated into the present application by reference,

- and mixtures thereof.

Preferably the polar oil is chosen from $C_{12}$-$C_{15}$ alcohol benzoate, diisopropyl sebacate, isopropyl lauroyl sarcosinate, dicaprylyl carbonate, 2-phenylethyl benzoate, butyloctyl salicylate, 2-octyldodecyl neopentanoate, dicaprylyl ether, isocetyl stearate, isodecyl neopentanoate, isononyl isononate, isopropyl myristate, isopropyl palmitate, isostearyl behenate, myristyl myristate, octyl palmitate and tridecyl trimellitate.

Preferably, the polar oil is a $C_{12}$-$C_{15}$ alkyl benzoate.

The mass content of polar oil (excluding lipophilic organic screening agent) relative to the total weight of the composition preferably ranges from 1% to 95% and more preferentially from 10% to 70% relative to the total weight of the composition.

The "fatty phase" of the compositions according to the invention may also comprise a wax, an apolar oil or mixtures thereof.

The term "wax" is understood to mean a compound which is solid or substantially solid at room temperature and which has a melting point generally of greater than 35°C.

The apolar oils and the waxes conventionally used in cosmetic compositions may be used in the compositions according to the present invention.

The compositions according to the invention may also comprise additional cosmetic and/or dermatological active agents.

A person skilled in the art will select said active agent(s) as a function of the effect desired on the skin, the hair, the eyelashes, the eyebrows and the nails.

Advantageously, the composition according to the invention also comprises at least one organic UV-screening agent.

Included among the organic UV-screening agents are in particular the following hydrophobic or hydrophilic UV-screening agents.

The term "hydrophobic screening agent" means:

- any screening agent that is lipophilic, i.e. which can be fully dissolved in molecular state in a liquid fatty phase or which can be dissolved in colloidal form (for example in micellar form) in a liquid fatty phase;
- any screening agent that is insoluble both in a liquid fatty phase and in a liquid aqueous phase.

The term "hydrophilic screening agent" means any screening agent that can be fully dissolved in molecular state in a liquid aqueous phase or that can be dissolved in colloidal form (for example in micellar form) in a liquid aqueous phase.

**Hydrophobic UV-A screening agents**

Dibenzoylmethane derivatives:

Butylmethoxydibenzoylmethane sold in particular under the trade name Parsol 1789 by DSM Nutritional Products, Inc.;
Isopropyldibenzoylmethane.

Aminobenzophenones:

n-Hexyl 2-(4-diethylamino-2-hydroxybenzoyl)benzoate sold in particular under the trade name Uvinul A+ by BASF;
1,1'-(1,4-Piperazinediyl)bis[1-[2-[4-(diethylamino)-2-hydroxybenzoyl]phenyl]methanone (CAS 919803-06-8).

Anthranilic derivatives:

Menthyl anthranilate sold in particular under the trade name Neo Heliopan MA by Symrise.

4,4-Diarylbutadiene derivatives:

1,1-Dicarboxy-(2,2'-dimethylpropyl)-4,4-diphenylbutadiene.

Merocyanin derivatives:

- Octyl 5-N,N-diethylamino-2-phenysulfonyl-2,4-pentadienoate.

In the context of the invention, and according to one particular embodiment, the following hydrophobic screening agents (A) are used:

- Butylmethoxydibenzoylmethane; and/or
- n-Hexyl2-(4-diethylamino-2-hydroxybenzoyl)benzoate.

**Hydrophilic UV-A screening agents**

- Terephthalylidenedicamphorsulfonic acid manufactured under the name Mexoryl SX by Chimex,

- Bis-benzazolyl derivatives as described in patents EP 669 323, and US 2 463 264 and more particularly the compound disodium phenyldibenzimidazotetrasulfonate sold under the trade name Neo Heliopan AP by Symrise.

The preferred water-soluble UV-A screening agent is terephthalylidenedicamphorsulfonic acid.

**Hydrophobic UV-B screening agents**

para-Am inobenzoates:

- Ethyl PABA;
- Ethyl dihydroxypropyl PABA;
- Ethylhexyl dimethyl PABA (Escalol 507 from ISP).

Salicylic derivatives:

Homosalate sold in particular under the name Eusolex HMS by Rona/EM Industries;
Ethylhexyl salicylate sold in particular under the name Neo Heliopan OS by Symrise;
Dipropylene glycol salicylate sold in particular under the name Dipsal by Scher; TEA salicylate sold under the name Neo Heliopan TS by Symrise.

Cinnamates

Ethylhexyl methoxycinnamate sold in particular under the trade name Parsol MCX by DSM Nutritional Products, Inc.;
Isopropyl methoxycinnamate;
Isoamyl methoxycinnamate sold in particular under the trade name Neo Heliopan E 1000 by Symrise;
Diisopropyl methylcinnamate;
Cinnoxate;
Glyceryl ethylhexanoate dimethoxycinnamate.

β,β'-Diphenylacrylate derivatives:

Octocrylene sold in particular under the trade name Uvinul N539 by BASF; Etocrylene sold in particular under the trade name Uvinul N35 by BASF.

Benzylidenecamphor derivatives:

3-Benzylidenecamphor manufactured under the name Mexoryl SD by Chimex; Methylbenzylidenecamphor sold in particular under the name Eusolex 6300 by Merck;
Polyacrylamidomethylbenzylidenecamphor manufactured under the name Mexoryl SW by Chimex.

Triazine derivatives:

Ethylhexyl triazone sold in particular under the trade name Uvinul T150 by BASF; Diethylhexylbutamidotriazone sold under the trade name Uvasorb HEB by Sigma 3V;
2,4,6-Tris(dineopentyl 4'-aminobenzalmalonate)-s-triazine;
2,4,6-Tris(diisobutyl 4'-aminobenzalmalonate)-s-triazine;
2,4-Bis(dineopentyl 4'-aminobenzalmalonate)-6-(n-butyl 4'-aminobenzoate)-s-triazine;
2,4-Bis(n-butyl 4'-aminobenzoate)-6-(aminopropyltrisiloxane)-s-triazine;

the symmetrical triazine screening agents described in patent US 6 225 467, patent application WO 2004/085 412 (see compounds 6 and 9) or the document Symmetrical Triazine Derivatives IP.COM Journal, IP.COM INC West Henrietta, NY, US (20 September 2004), especially 2,4,6-tris(biphenyl)-1,3,5-triazines (in particular 2,4,6-tris(biphenyl-4-yl-1,3,5-triazine) and 2,4,6-tris(terphenyl)-1,3,5-triazine, the latter two screening agents being described in Beiersdorf patent applications WO 06/035 000, WO 06/034 982, WO 06/034 991, WO 06/035 007, WO 2006/034 992 and WO 2006/034 985).

Imidazoline derivatives:

Ethylhexyl dimethoxybenzylidene dioxoimidazoline propionate.

Benzalmalonate derivatives:

Polyorganosiloxanes containing benzalmalonate functions, for instance Polysilicone-15, sold in particular under the trade name Parsol SLX by DSM Nutritional Products, Inc.;
Dineopentyl 4'-methoxybenzalmalonate.

Within the context of the invention, and according to one particular embodiment, the following hydrophobic UV-B screening agents are used in the composition of the invention:

- Ethylhexyl salicylate;
- Octocrylene;
- Ethylhexyl triazone.

**Hydrophilic UV-B screening agents**

The following p-aminobenzoic acid (PABA) derivatives: PABA, Glyceryl PABA and PEG-25 PABA sold in particular under the trade name Uvinul P25 by BASF. Phenylbenzimidazolesulfonic acid sold in particular under the trade name Eusolex 232 by Merck, ferulic acid, salicylic acid, DEA methoxycinnamate, benzylidenecamphorsulfonic acid manufactured under the name Mexoryl SL by Chimex, camphorbenzalkonium methosulfate manufactured under the name Mexoryl SO by Chimex.
The preferred UVB screening agent is phenylbenzimidazolesulfonic acid.

**Mixed UVA and UVB hydrophobic screening agents**

Benzophenone derivatives:

Benzophenone-1 sold in particular under the trade name Uvinul 400 by BASF;
Benzophenone-2 sold in particular under the trade name Uvinul D50 by BASF;
Benzophenone-3 or oxybenzone sold in particular under the trade name Uvinul M40 by BASF;
Benzophenone-6 sold in particular under the trade name Helisorb 11 by Norquay;
Benzophenone-8 sold in particular under the trade name Spectra-Sorb UV-24 by American Cyanamid;
Benzophenone-10;
Benzophenone-11;
Benzophenone-12.

Phenylbenzotriazole derivatives:

- Drometrizole trisiloxane sold in particular under the name Silatrizole by Rhodia Chimie or manufactured under the name Meroxyl XL by the company Chimex;
- Methylenebis(benzotriazolyl)tetramethylbutylphenol sold in solid form in particular under the trade name Mixxim BB/100 by Fairmount Chemical, or in micronized form as an aqueous dispersion in particular under the trade name

Tinosorb M by Ciba Specialty Chemicals.

Bis-resorcinyl triazine derivatives:

Bis(ethylhexyloxyphenol)methoxyphenyltriazine sold in particular under the trade name Tinosorb S by Ciba-Geigy.

Benzoxazole derivatives:

2,4-Bis[5-(1-dimethylpropyl)benzoxazol-2-yl-(4-phenyl)imino]-6-(2-ethylhexyl)imino-1,3,5-triazine, sold in particular under the name Uvasorb K2A by Sigma 3V.
The preferred UVA and UVB hydrophobic organic screening agents are chosen from:

- Drometrizole trisiloxane;
- Methylenebis(benzotriazolyl)tetramethylbutylphenol;
- Bis(ethylhexyloxyphenol)methoxyphenyltriazine.

**Mixed UVA and UVB hydrophilic screening agents**

Benzophenone derivatives comprising at least one sulfonic radical, such as

Benzophenone-4 sold in particular under the trade name Uvinul MS 40 by BASF,
Benzophenone-5 and
Benzophenone-9.

The organic screening agents, when they are present, are present in contents ranging from 0.01% to 30% by weight and preferably from 0.1 % to 20% by weight relative to the total weight of the composition of the invention.

**ADDITIVES**

The compositions in accordance with the present invention may also comprise standard cosmetic adjuvants chosen especially from organic solvents, ionic or nonionic, hydrophilic or lipophilic thickeners, softeners, humectants, opacifiers, stabilizers, emollients, silicones, antifoams, fragrances, preserving agents, anionic, cationic, nonionic, zwitterionic or amphoteric surfactants, fillers, polymers, propellants, basifying or acidifying agents or any other ingredient commonly used in the cosmetics and/or dermatological field.
Among the organic solvents that may be mentioned are lower alcohols and polyols. These polyols may be chosen from glycols and glycol ethers, for instance ethylene glycol, propylene glycol, butylene glycol, dipropylene glycol or diethylene glycol.
Hydrophilic thickeners that may be mentioned include carboxyvinyl polymers, such as Carbopols (Carbomers) and the Pemulens (acrylate/C10-C30 alkyl acrylate copolymer); polyacrylamides, for instance the crosslinked copolymers sold under the names Sepigel 305 (CTFA name: polyacrylamide/C13-C14 isoparaffin/Laureth 7) or Simulgel 600 (CTFA name: acrylamide/sodium acryloyldimethyl taurate copolymer/isohexadecane/polysorbate 80) by the company SEPPIC; 2-acrylamido-2-methylpropanesulfonic acid polymers and copolymers, optionally crosslinked and/or neutralized, for instance poly(2-acrylamido-2-methylpropanesulfonic acid) sold by the company Clariant under the trade name Hostacerin AMPS (CTFA name: ammonium polyacryloyldimethyl taurate) or Simulgel 800 sold by the company SEPPIC (CTFA name: sodium polyacryloyldimethyl taurate/polysorbate 80/sorbitan oleate); copolymers of 2-acrylamido-2-methylpropanesulfonic acid and of hydroxyethyl acrylate, for instance Simulgel NS and Sepinov EMT 10 sold by the company SEPPIC; cellulose derivatives such as hydroxyethyl cellulose; polysaccharides and especially gums such as xanthan gum; and mixtures thereof.
Lipophilic thickeners that may be mentioned include synthetic polymers, such as the poly(C10-C30 alkyl acrylates) sold under the names Intelimer IPA 13-1 and Intelimer IPA 13-6 by the company Landec, or else modified clays, such as hectorite and its derivatives, for instance the products sold under the name Bentone.
The compositions according to the invention may also comprise additional cosmetic or dermatological active agents.
Mention may be made, among active agents, of:

- vitamins (A, C, E, K, PP, and the like) and their derivatives or precursors, alone or as mixtures;
- antioxidants;

- free-radical scavengers;
- antiglycation agents;
- calmatives;
- NO-synthase inhibitors;
- agents for stimulating the synthesis of dermal or epidermal macromolecules and/or for preventing their degradation;
- agents for stimulating fibroblast proliferation;
- agents for stimulating keratinocyte proliferation;
- muscle relaxants;
- tensioning agents;
- matting agents;
- keratolytic agents;
- desquamating agents;
- moisturizers, for instance polyols such as glycerol, butylene glycol or propylene glycol;
- antiinflammatory agents;
- agents that act on the energy metabolism of cells;
- insect repellents;
- substance P or substance CRGP antagonists;
- hair-loss counteractants and/or hair restorers;
- anti-wrinkle agents.

Needless to say, a person skilled in the art will take care to select the aforementioned optional additional compound(s) and/or the amounts thereof such that the advantageous properties intrinsically associated with the compositions in accordance with the invention are not, or are not substantially, adversely affected by the envisaged addition(s).
A person skilled in the art will select the said active agent(s) as a function of the effect desired on the skin, the hair, the eyelashes, the eyebrows and the nails.

## Galenical forms

The compositions according to the invention may be prepared according to the techniques that are well known to those skilled in the art.
The compositions according to the invention are in the form of an oil-in-water emulsion and may have the appearance of a milk, a cream or a cream gel. They may optionally be packaged as an aerosol and may be in the form of a spray.
The emulsification processes that may be used are of the paddle or impeller, rotor-stator or HPH type.
To obtain stable emulsions with a low content of emulsifying compound (oil/emulsifier ratio > 25), it is possible to make the dispersion in concentrated phase and then to dilute the dispersion with the rest of the aqueous phase.
It is also possible, via HPH (between 50 and 800 bar), to obtain stable dispersions with drop sizes that may be as small as 100 nm.
The emulsions generally contain at least one emulsifier chosen from amphoteric, anionic, cationic or nonionic emulsifiers, used alone or as a mixture. The emulsions may also contain stabilizers of other types, for instance fillers, or gelling or thickening polymers.
As emulsifying surfactants that may be used for the preparation of the O/W emulsions, examples that may be mentioned include nonionic emulsifiers such as oxyalkylenated (more particularly polyoxyethylenated) esters of fatty acids and of glycerol; oxyalkylenated esters of fatty acids and of sorbitan; oxyalkylenated (oxyethylenated and/or oxypropylenated) esters of fatty acids, such as the PEG 100 stearate/glyceryl stearate mixture sold, for example, by ICI under the name Arlacel 165; oxyalkylenated (oxyethylenated and/or oxypropylenated) ethers of fatty alcohols; esters of sugars, such as sucrose stearate; or ethers of fatty alcohol and of sugar, in particular alkyl polyglucosides (APGs), such as decyl glucoside and lauryl glucoside, sold, for example, by the company Henkel under the respective names Plantaren 2000 and Plantaren 1200, cetostearyl glucoside, optionally as a mixture with cetostearyl alcohol, sold, for example, under the name Montanov 68 by the company SEPPIC, under the name Tegocare CG90 by the company Goldschmidt and under the name Emulgade KE3302 by the company Henkel, and also arachidyl glucoside, for example in the form of a mixture of arachidyl alcohol, behenyl alcohol and arachidyl glucoside, sold under the name Montanov 202 by the company SEPPIC. According to one particular embodiment of the invention, the mixture of the alkyl polyglucoside as defined above with the corresponding fatty alcohol may be in the form of a self-emulsifying composition, for example as described in document WO-A-92/06778.
According to one particular embodiment, the composition comprises at least one emulsifier chosen from dimer surfactants known as "gemini surfactants" comprising two identical or different surfactant units, each formed from a hydrophilic head and a hydrophobic tail and linked together, via the hydrophilic heads, by a spacer group. Such surfactants

are especially described in patent applications DE 19943681, DE 19943668, DE 42 27 391 and DE 196 08 117; JP-A-11-60437; JP-A-8-311003; EP 0 697 244; EP 0 697 245; EP 0 708 079; DE 19622612 and JP-A 10-17593; WO 03/024412; US 5863886; WO 96/25388; WO 96/14926; WO 96/16930; WO 96/25384; WO 97/40124; WO 97/31890; DE 19750246; DE 19750245; DE 19631225; DE 19647060. For a detailed description of the various chemical structures and of their physicochemical properties, reference may be made to the following publications:

Milton J. Rosen, Gemini Surfactants, Properties of surfactant molecules with two hydrophilic groups and two hydrophobic groups, Cosmetics & Toiletries magazine, vol. 113, December 1998, pages 49-55,
Milton J. Rosen, Recent Developments in Gemini Surfactants, Allured's Cosmetics & Toiletries magazine, July 2001, vol. 116, No. 7, pages 67-70.

Preferred in particular among the above dimer surfactants are the anionic surfactants corresponding to formula (I):

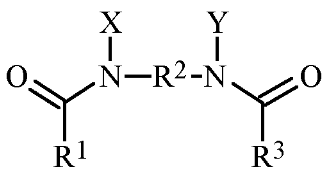

(I)

where

$R^1$ and $R^3$ represent a linear $C_8$-$C_{16}$ alkyl group,
$R^2$ represents a $C_2$-$C_8$ alkylene group,
X and Y each represent a group $(C_2H_4O)_x$-RF with x = 10-15, and RF = $-SO_3M$ where M represents an alkali metal atom.

A preferred gemini surfactant of this family is a sodium dicocoylethylenediamine PEG-15 sulfate (INCI name) anionic compound of formula:

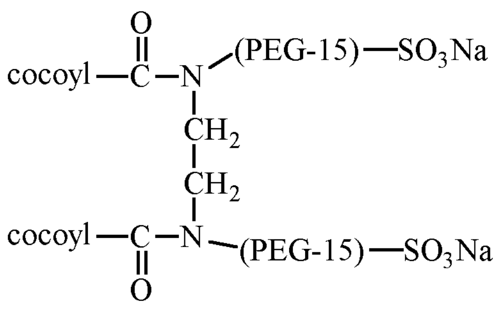

This gemini surfactant may be used, for example, in the following mixtures sold by the company Sasol under the name Ceralution®:

- Ceralution® H: Behenyl Alcohol, Glyceryl Stearate, Glyceryl Stearate Citrate and Sodium Dicocoylethylenediamine PEG-15 Sulfate,
- Ceralution® F: Sodium Lauroyl Lactylate and Sodium Dicocoylethylenediamine PEG-15 Sulfate,
- Ceralution® C: Aqua, Capric/Caprylic triglyceride, Glycerin, Ceteareth-25, Sodium Dicocoylethylenediamine PEG-15 Sulfate, Sodium Lauroyl Lactylate, Behenyl Alcohol, Glyceryl Stearate, Glyceryl Stearate Citrate, Gum Arabic, Xanthan Gum, Phenoxyethanol, Methylparaben, Ethylparaben, Butylparaben, Isobutylparaben (INCI names).

The mixture Behenyl Alcohol, Glyceryl Stearate, Glyceryl Stearate Citrate and Sodium Dicocoylethylenediamine PEG-15 Sulfate (Ceralution® H) will be used more particularly.
The concentration of gemini surfactant(s) used in the present invention preferably ranges from 0.001% to 8%, preferably from 0.01% to 4% and in particular from 0.05% to 3% relative to the total weight of the photoprotective composition.
Among the other emulsion stabilizers, use may also be made of isophthalic acid or sulfoisophthalic acid polymers,

and in particular phthalate/sulfoisophthalate/glycol copolymers, for example the diethylene glycol/phthalate/isophthalate/1,4-cyclohexanedimethanol copolymer (INCI name: Polyester-5) sold under the name Eastman AQ Polymer (AQ35S, AQ38S, AQ55S and AQ48 Ultra) by the company Eastman Chemical.

Among the other emulsion stabilizers, mention may also be made of hydrophobic modified 2-acrylamido-2-methylpropanesulfonic acid polymers such as those described in patent application EP 1 069 142.

When it is an emulsion, the aqueous phase of this emulsion may comprise a nonionic vesicular dispersion prepared according to known processes (Bangham, Standish and Watkins, J. Mol. Biol. 13, 238 (1965), FR 2 315 991 and FR 2 416 008).

## Examples

Compositions 1 to 3 below were prepared. The ingredients are given as weight percentages of active material relative to the total weight of the composition.

| Phase | Ingredients | Ex. 1 outside the invention | Ex. 2 invention | Ex. 3 invention |
|---|---|---|---|---|
| A | Glycerol | 5 | 5 | 5 |
| | EDTA | 0.1 | 0.1 | 0.1 |
| | Styrene/Acrylates Copolymer (Sunspheres Powder - Rohm & Haas) | 2 | 2 | 2 |
| | Deionized water | qs 100 | qs 100 | qs 100 |
| | Triethanolamine | 0.3 | 0.3 | 0.3 |
| | Preserving agents | 1.2 | 1.2 | 1.2 |
| | | | | |
| B1 | C12-15 alkyl benzoate | 4 | 4 | 4 |
| | Preserving agents | 0.25 | 0.25 | 0.25 |
| | Isononyl isononanoate | 2 | 2 | 2 |
| | Ceralution H | 3 | 3 | 3 |
| | Cetyl alcohol | 0.5 | 0.5 | 0.5 |
| | Diisopropyl sebacate | 4 | 4 | 4 |
| | 2-Ethylhexyl $\alpha$-cyano-($\beta$,$\beta$'-diphenylacrylate | 3.5 | 3.5 | 3.5 |
| | 2,4,6-Tris[p-(2'-ethylhexyl-1'-oxycarbonyl)anilino]-1,3,5-triazine | 2.3 | 2.3 | 2.3 |
| | 4-*tert*-Butyl-4'-methoxydibenzoylmethan e | 3 | 3 | 3 |
| | Drometrizole trisiloxane | 0.5 | 0.5 | 0.5 |
| | Cyclohexadimethylsiloxa ne | 2 | 2 | 2 |
| | Poly(C10-30)alkyl acrylate | 2 | 2 | 2 |
| | | | | |
| B2 | Hostacerin AMPS | 0.5 | 0.5 | 0.5 |
| | Crosslinked polyacrylate | 2 | 2 | 2 |
| | | | | |
| C | Eospoly TR | 2.5 | 2.5 | 2.5 |
| | Silseem Mistypearl Yellow | 0 | 0 | 2.5 |
| | TTC 30 | 0 | 2.5 | 0 |

These compositions were evaluated according to the following properties: Stability

Screening efficacy, and

Evaluation of the glidance after application to the skin

Emulsion preparation method:

The aqueous phase A and oily phase $B_1$ are prepared by mixing the raw materials, with mechanical stirring, at 80°C; the solutions obtained are macroscopically homogeneous. The emulsion is prepared by slow introduction of the oily phase into the aqueous phase with stirring using a Moritz homogenizer at a stirring speed of 4000 rpm for 15 minutes. The emulsion obtained is cooled, with stirring, to 40°C, then the oily phase $B_2$ is added thereto with gentle stirring, followed by the phase C. The emulsion obtained is cooled to room temperature with gentle stirring. It is characterized by drops between 1 μm and 10 μm in size.

***In vitro* protocol for evaluating the screening efficacy**

The sun protection factor (SPF) is determined according to the *"in vitro"* method described by B.L. Diffey in J. Soc. Cosmet. Chem. 40, 127-133, (1989). The measurements were made using a UV-1000S spectrophotometer from the company Labsphere. Each composition is applied to a rough plate of PMMA, in the form of a homogeneous and even deposit at a rate of 1 $mg/cm^2$.

**Protocol for evaluating the stability of the compositions of the invention**

The stability of the compositions of the invention is evaluated by macroscopic and microscopic observations of their aspect and by measuring their viscosity using a Rheomat 180 viscometer at 25°C at a shear rate of 200 $s^{-1}$ and with a spindle chosen as a function of the viscosity of the composition. A composition is judged stable when its macroscopic and microscopic aspect and its viscosity are stable for 1 month at room temperature.

**Protocol for evaluating the glidance after application to the skin**

The glidance after application of the formula to the skin is evaluated by applying the formula to a forearm at a rate of 2 $mg/cm^2$, waiting for a drying time equal to 2 minutes and then assessing the friction force felt between the fingers and the surface of the forearm.

**Results**

TABLE I

| Examples | *in vitro* SPF | Stability | Glidance |
|---|---|---|---|
| **Example 1 (outside the invention)** | 78 | - | - |
| **Example 2** | 112 | ++ | +++ |
| **Example 3** | 106 | + | +++ |

These results show that the mixture of spherical particles of composite material and of platelet-shaped particles of composite material according to the invention make it possible to obtain a highly efficient screening system with a low content of screening agents, which shows good stability and good cosmeticity. For the composition comprising a screening composite material of spherical shape (Example 1), the performance qualities in terms of screening efficacy, stability or cosmeticity are inferior to those of the combination of the invention.

**Claims**

**1.** Composition in the form of an oil-in-water emulsion containing, in a cosmetically acceptable medium, a mixture of

composite particles comprising:

a) at least spherical composite particles A with a mean size of between 0.1 and 30 μm comprising a matrix and an inorganic UV-screening agent, the content of inorganic screening agent in a particle ranging from 1% to 70% by weight,
b) at least non-spherical composite particles B with a mean size of between 0.3 and 30 μm comprising a matrix and an inorganic UV-screening agent, the content of inorganic screening agent in a particle ranging from 1% to 70% by weight.

**2.** Composition according to Claim 1, in which the matrix of the spherical composite particles and that of the non-spherical particles comprise one or more organic and/or inorganic materials.

**3.** Composition according to Claim 1 or 2, in which the spherical composite particles and/or the non-spherical composite particles are formed from a matrix comprising an organic and/or inorganic material, in which particles of inorganic UV-screening agent are included.

**4.** Composition according to Claim 1 or 2, in which the spherical composite particles and/or the non-spherical composite particles contain a matrix made of an organic and/or inorganic material, covered with at least one layer of inorganic UV-screening agent which may be connected to the matrix by means of a binder.

**5.** Cosmetic composition according to either of Claims 1 and 2, in which the spherical composite particles and/or the non-spherical composite particles contain an inorganic UV-screening agent covered with at least one layer of an organic and/or inorganic material.

**6.** Composition according to any one of Claims 1 to 5, in which the inorganic UV-screening agent is chosen from metal oxides, in particular from titanium, zinc or iron oxides and more particularly titanium dioxide ($TiO_2$).

**7.** Composition according to any one of Claims 1 to 6, in which the inorganic material used in the matrix of the spherical composite particles is chosen from the group formed by glass, silica and aluminium oxide, and mixtures thereof.

**8.** Composition according to any one of Claims 1 to 7, in which the organic material used in the matrix of the spherical composite particles is chosen from the group formed by poly(meth)acrylates, polyamides, silicones, polyurethanes, polyethylenes, polypropylenes, polystyrenes, polyhydroxyalkanoates, polycaprolactams, poly(butylene)succinates, polysaccharides, polypeptides, polyvinyl alcohols, polyvinyl resins, fluoropolymers, waxes, polyesters, polyethers, and mixtures thereof.

**9.** Composition according to any one of Claims 1 to 8, in which the matrix of the spherical composite particles contains a material or mixture of materials chosen from:

- $SiO_2$,
- PMMA,
- copolymers of styrene and of a C1/C5 alkyl acrylate,
- polyamides, such as nylon.

**10.** Composition according to any one of Claims 1 to 9, in which the organic materials constituting the matrix are chosen from:

- triethoxycaprylylsilane,
- polymethyl methacrylate and acrylic copolymers comprising other types of monomer such as styrene,
- polyamides, such as nylon.

**11.** Composition according to any one of Claims 1 to 10, in which the inorganic materials constituting the matrix of the non-spherical composite particles are chosen from:

- silica,
- talc,
- mica,
- alumina.

**12.** Composition according to any one of Claims 1 to 11, in which the matrix of the non-spherical composite particles contains a material or mixture of materials chosen from:

- alumina,
- an alumina/triethoxycaprylylsilane mixture,
- talc,
- silica,
- mica.

**13.** Composition according to any one of Claims 1 to 12, in which the non-spherical composite particles are platelet-shaped.

**14.** Composition according to any one of Claims 1 to 13, comprising at least one polar oil which preferably has a surface tension of greater than or equal to 10 mN/m at 25°C and at atmospheric pressure and more particularly a $C_{12}$-$C_{15}$ alkyl benzoate.

**15.** Composition according to any one of Claims 1 to 14, also containing an organic UV-screening agent.

**Patentansprüche**

**1.** Zusammensetzung in Form einer Öl-in-Wasser-Emulsion, die in einem kosmetisch annehmbaren Medium eine Mischung von Verbundpartikeln enthält, umfassend:

a) mindestens kugelförmige Verbundpartikel A mit einer mittleren Größe zwischen 0,1 und 30 μm, umfassend eine Matrix und ein anorganisches UV-Schutzmittel, wobei der Gehalt an anorganischem Schutzmittel in einem Partikel im Bereich von 1 Gew.% bis 70 Gew.% liegt,
b) mindestens nicht-kugelförmige Verbundpartikel B mit einer mittleren Größe zwischen 0,3 und 30 μm, umfassend eine Matrix und ein anorganisches UV-Schutzmittel, wobei der Gehalt an anorganischem Schutzmittel in einem Partikel im Bereich von 1 Gew.% bis 70 Gew.% liegt.

**2.** Zusammensetzung nach Anspruch 1, wobei die Matrix der kugelförmigen Verbundpartikel und diejenige der nicht-kugelförmigen Partikel ein oder mehrere organische und/oder anorganische Materialien umfassen.

**3.** Zusammensetzung nach Anspruch 1 oder 2, wobei die kugelförmigen Verbundpartikel und/oder die nicht-kugelförmigen Verbundpartikel aus einer Matrix gebildet sind, umfassend ein organisches und/oder anorganisches Material, wobei Partikel des anorganischen UV-Schutzmittels eingeschlossen sind.

**4.** Zusammensetzung nach Anspruch 1 oder 2, wobei die kugelförmigen Verbundpartikel und/oder die nicht-kugelförmigen Verbundpartikel eine Matrix enthalten, die aus organischem und/oder anorganischem Material hergestellt ist, bedeckt mit mindestens einer Schicht des anorganischen UV-Schutzmittels, das mithilfe eines Bindemittels mit der Matrix verbunden sein kann.

**5.** Kosmetische Zusammensetzung nach einem der Ansprüche 1 und 2, wobei die kugelförmigen Verbundpartikel und/oder die nicht-kugelförmigen Verbundpartikel ein anorganisches UV-Schutzmittel enthalten, das mit mindestens einer Schicht aus organischem und/oder anorganischem Material bedeckt ist.

**6.** Zusammensetzung nach einem der Ansprüche 1 bis 5, wobei das anorganische UV-Schutzmittel ausgewählt ist aus Metalloxiden, insbesondere aus Titan-, Zink- oder Eisenoxiden und insbesondere Titandioxid ($TiO_2$).

**7.** Zusammensetzung nach einem der Ansprüche 1 bis 6, wobei das in der Matrix der kugelförmigen Verbundpartikel verwendete anorganische Material ausgewählt ist aus der Gruppe, die durch Glas, Siliciumdioxid und Aluminiumoxid und Mischungen davon gebildet ist.

**8.** Zusammensetzung nach einem der Ansprüche 1 bis 7, wobei das in der Matrix der kugelförmigen Verbundpartikel verwendete organische Material ausgewählt ist aus der Gruppe, die durch Poly(meth)acrylate, Polyamide, Silikone, Polyurethane, Polyethylene, Polypropylene, Polystyrole, Polyhydroxyalkanoate, Polycaprolactame, Poly(butylen)succinate, Polysaccharide, Polypeptide, Polyvinylalkohole, Polyvinylharze, Fluorpolymere, Wachse, Polyester,

Polyether und Mischungen davon gebildet ist.

9. Zusammensetzung nach einem der Ansprüche 1 bis 8, wobei die Matrix der kugelförmigen Verbundpartikel ein Material oder eine Mischung von Materialien enthält, die ausgewählt sind aus:

   - $SiO_2$,
   - PMMA,
   - Copolymeren von Styrol und $C_1/C_5$-Alkylacrylat,
   - Polyamiden, wie Nylon.

10. Zusammensetzung nach einem der Ansprüche 1 bis 9, wobei die organischen Materialien, die die Matrix stellen, ausgewählt sind aus:

    - Triethoxycaprylylsilan,
    - Polymethylmethacrylat und Acrylcopolymeren, die andere Monomertypen umfassen, wie Styrol,
    - Polyamiden, wie Nylon.

11. Zusammensetzung nach einem der Ansprüche 1 bis 10, wobei die anorganischen Materialien, die die Matrix der nicht-kugelförmigen Verbundpartikel stellen, ausgewählt sind aus:

    - Siliciumdioxid,
    - Talkum,
    - Glimmer,
    - Aluminiumoxid.

12. Zusammensetzung nach einem der Ansprüche 1 bis 11, wobei die Matrix der nicht-kugelförmigen Verbundpartikel ein Material oder eine Mischung von Materialien enthält, die ausgewählt sind aus:

    - Aluminumoxid,
    - einer Aluminiumoxid-/Triethoxycaprylylsilan-Mischung,
    - Talkum,
    - Siliciumdioxid,
    - Glimmer.

13. Zusammensetzung nach einem der Ansprüche 1 bis 12, wobei die nicht-kugelförmigen Verbundpartikel plättchenförmig sind.

14. Zusammensetzung nach einem der Ansprüche 1 bis 13, umfassend mindestens ein polares Öl, das vorzugsweise eine Oberflächenspannung größer als oder gleich 10 mN/m bei 25 °C und atmosphärischem Druck hat und insbesondere ein $C_{12}$-$C_{15}$-Alkylbenzoat umfasst.

15. Zuaammensetzung nach einem der Ansprüche 1 bis 14, die auch ein organisches UV-Schutzmittel enthält.

## Revendications

1. Composition sous forme d'émulsion huile-dans-eau contentant dans un milieu cosmétiquement acceptable un mélange de particules composites comprenant :

   a) au moins des particules A composites sphériques de taille moyenne comprise entre 0,1 et 30 µm comprenant une matrice et un filtre UV inorganique, la teneur en filtre inorganique dans une particule allant de 1 % à 70 % en poids ;
   b) au moins des particules B composites non-sphériques de taille moyenne comprise entre 0,3 et 30 µm comprenant une matrice et un filtre UV inorganique, la teneur en filtre inorganique dans une particule allant de 1 % à 70 % en poids.

2. Composition selon la revendication 1, dans laquelle la matrice des particules composites sphériques et celle des particules non sphériques comprennent un ou plusieurs matériaux organiques et/ou inorganiques.

3. Composition selon la revendication 1 ou 2, dans laquelle les particules composites sphériques et/ou les particules composites non-sphériques sont constituées d'une matrice comprenant un matériau organique et /ou inorganique dans laquelle sont incluses des particules de filtre UV inorganique.

4. Composition selon la revendication 1 ou 2, dans laquelle les particules composites sphériques et/ou les particules composites non-sphériques contiennent une matrice en un matériau organique et/ou inorganique recouverte d'au moins une couche de filtre UV inorganique pouvant être connectée à la matrice à l'aide d'un liant.

5. Composition cosmétique selon l'une quelconque des revendications 1 ou 2, dans laquelle les particules composites sphériques et/ou les particules composites non sphériques contiennent un filtre UV inorganique recouvert d'au moins une couche d'un matériau organique et/ou inorganique.

6. Composition selon l'une quelconque des revendications 1 à 5, dans laquelle le filtre UV inorganique est choisi parmi les oxydes métalliques en particulier parmi les oxydes de titane, zinc ou fer et plus particulièrement le dioxyde de titane ($TiO_2$).

7. Composition selon l'une quelconque des revendications 1 à 6, dans laquelle le matériau inorganique utilisé dans la matrice des particules composites sphériques est choisi dans le groupe formé par le verre, la silice, l'oxyde d'aluminium et leurs mélanges.

8. Composition selon l'une quelconque des revendications 1 à 7, dans laquelle le matériau organique utilisé dans la matrice des particules composites sphériques est choisi dans le groupe formé par les poly(méth)acrylates, polyamides, silicones, polyuréthanes, polyéthylènes, polypropylènes, polystyrènes, polyhydroxyalkanoates, polyrcaprolactames, poly(butylène)succinates, polysaccharides, polypeptides, polyvinylalcools, résines polyvinyliques, fluoropolymères, cires, polyesters, polyéthers, et leurs mélanges.

9. Composition selon l'une quelconque des revendications 1 à 8, dans laquelle la matrice des particules composites sphériques continent un matériau ou un mélange de matériaux choisi parmi :

- le $SiO_2$,
- le PMMA,
- les copolymères de styrène et d'un acrylate d'alkyle en C1/C5,
- les polyamides tels que le nylon.

10. Composition selon l'une quelconque des revendications 1 à 9, dans laquelle les matériaux organiques constituant la matrice sont choisis parmi :

- le triéthoxycaprylylsilane,
- le polyméthacrylate de méthyle et les copolymères acryliques comprenant d'autres types de monomères tels que le styrène,
- les polyamides tels que le nylon.

11. Composition selon l'une quelconque des revendications 1 à 10, dans laquelle les matériaux inorganiques constituant la matrice des particules composites non-sphériques sont choisis parmi :

- la silice,
- le talc,
- le mica,
- l'alumine.

12. Composition selon l'une quelconque des revendications 1 à 11, dans laquelle la matrice des particules composites non-sphériques continent un matériau ou un mélange de matériaux choisis parmi :

- l'alumine,
- le mélange alumine/triéthoxycaprylylsilane,
- le talc,
- la silice,
- le mica.

13. Composition selon l'une quelconque des revendications 1 à 12, dans laquelle les particules composites non-sphériques sont plaquettaires.

14. Composition selon l'une quelconque des revendications 1 à 13, comprenant au moins une huile polaire ayant de préférence une tension de surface supérieure ou égale à 10 mN/m à 25°C et sous pression atmosphérique et plus particulièrement un benzoate d'alkyle en $C_{12}$-$C_{15}$.

15. Composition selon l'une quelconque des revendications 1 à 14, contentant en plus un filtre UV organiques.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- FR 2882371
- WO 2006083326 A
- WO 9837964 A
- WO 2006061835 A
- EP 1388550 A
- WO 9822539 A
- US 2008188574 A
- JP 2007302647 A
- FR 0853634
- EP 0955039 A
- US 2004175338 A
- EP 669323 A
- US 2463264 A
- US 6225467 B
- WO 2004085412 A
- WO 06035000 A
- WO 06034982 A
- WO 06034991 A
- WO 06035007 A
- WO 2006034992 A
- WO 2006034985 A
- WO 9206778 A
- DE 19943681
- DE 19943668
- DE 4227391
- DE 19608117
- JP 11060437 A
- JP 8311003 A
- EP 0697244 A
- EP 0697245 A
- EP 0708079 A
- DE 19622612
- JP 10017593 A
- WO 03024412 A
- US 5863886 A
- WO 9625388 A
- WO 9614926 A
- WO 9616930 A
- WO 9625384 A
- WO 9740124 A
- WO 9731890 A
- DE 19750246
- DE 19750245
- DE 19631225
- DE 19647060
- EP 1069142 A
- FR 2315991
- FR 2416008


### Non-patent literature cited in the description


- **C.M. HANSEN.** The three dimensional solubility parameters. *J. Paint Technol.,* 1967, vol. 39, 105
- **MILTON J. ROSEN.** Gemini Surfactants, Properties of surfactant molecules with two hydrophilic groups and two hydrophobic groups. *Cosmetics & Toiletries magazine,* December 1998, vol. 113, 49-55
- **MILTON J. ROSEN.** Recent Developments in Gemini Surfactants. *Allured's Cosmetics & Toiletries magazine,* July 2001, vol. 116 (7), 67-70
- **BANGHAM ; STANDISH ; WATKINS.** *J. Mol. Biol.,* 1965, vol. 13, 238
- **B.L. DIFFEY.** *J. Soc. Cosmet. Chem.,* 1989, vol. 40, 127-133